(19) Europäisches Patentamt
European Patent Office
Office européen des brevets

(11) Veröffentlichungsnummer: **0 258 787 B1**

(12) **EUROPÄISCHE PATENTSCHRIFT**

(45) Veröffentlichungstag der Patentschrift: **04.03.92**

(51) Int. Cl.[5]: **C11B 9/00**, A61K 7/46

(21) Anmeldenummer: **87112312.1**

(22) Anmeldetag: **25.08.87**

# (54) Verwendung von 2-tert.-Butyl-4-methyl-cyclohexanol als Riechstoff sowie als Bestandteil von Riechstoffkompositionen.

(30) Priorität: **30.08.86 DE 3629605**

(43) Veröffentlichungstag der Anmeldung:
**09.03.88 Patentblatt 88/10**

(45) Bekanntmachung des Hinweises auf die Patenterteilung:
**04.03.92 Patentblatt 92/10**

(84) Benannte Vertragsstaaten:
**CH DE ES FR GB LI NL**

(56) Entgegenhaltungen:
**CH-A- 620 099**
**FR-A- 2 300 752**

**JOURNAL OF THE AMERICAN CHEMICAL SOCIETY, Band 66, Januar 1944, Seiten 118-122; H.E. UNGNADE et al.: "The preparation of cyclohexanols by catalytic reduction of phenols"**

(73) Patentinhaber: **BASF Aktiengesellschaft**
**Carl-Bosch-Strasse 38**
**W-6700 Ludwigshafen(DE)**

(72) Erfinder: **Gramlich, Walter, Dr.**
**Auf der Hoehe 11**
**W-6803 Edingen-Neckarhausen(DE)**
Erfinder: **Hoffmann, Werner, Dr.**
**14 Horatio Street, Apt. 11D.**
**New York, N.Y. 10014(US)**
Erfinder: **Jansen, Klaas**
**Tannenstrasse 30**
**W-6700 Ludwigshafen(DE)**
Erfinder: **Lengsfeld, Wolfgang, Dr.**
**Woogstrasse 46**
**W-6703 Limburgerhof(DE)**
Erfinder: **Schuster, Ludwig, Dr.**
**Weinheimer Strasse 44**
**W-6703 Limburgerhof(DE)**

EP 0 258 787 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Auf dem Gebiet der Parfümerie und der Aromaentwicklung besteht trotz der großen Zahl der bereits bekannten natürlichen und synthetischen Riechstoffe nach wie vor ein großer Bedarf an neuen Riechstoffen, die entweder bisher unbekannte Geruchsnoten aufweisen oder aber teure bzw. schwierig zugängliche Riechstoffe ersetzen können. Ursache ist der ständig wachsende Bedarf an Parfümkompositionen, sowohl für die Feinparfümerie als auch für kosmetische und technische Produkte wie Waschmittel, Weichspüler u. dgl.

Es wurde nun gefunden, daß 2-tert.-Butyl-4-methyl-cyclohexanol (I) einen hochinteressanten, vielseitigen, insbesondere sowohl im sauren als auch in alkalischen Medien einsetzbaren Riechstoff von erdig-holzigem Vetiver-artigem Geruch darstellt, der sich zudem durch große Geruchsintensität auszeichnet.

I sowie dessen Synthese ist in der Literatur bereits von H. Ungnade und A. D. McLaren im J. Amer. Chem. Soc., Vol. 66, (1944) Seiten 118 - 124 in einem Artikel über die katalytische Hydrierung von Phenolen beschrieben. Die Autoren geben jedoch keinerlei Hinweise auf die interessanten Geruchseigenschaften dieser Verbindung. Darüber hinaus ist keine Beschreibung von I bekannt.

Man erhält I auf einfache Weise durch Hydrieren des entsprechenden Phenols.

Die Kernhydrierung von Phenolen erfolgt in an sich bekannter Weise in Gegenwart bekannter Hydrierkatalysatoren wie Raney-Nickel, Palladium-, Rhodium- oder Ruthenium-Katalysatoren.

Gemäß loc. cit. wurde Raney-Nickel als Katalysator eingesetzt. Wir erzielten mit Rutheniumkatalysatoren die besten Ausbeuten.

Je nach den angewandten Reakionsbedingungen erhält man unterschiedliche Diastereomerengemische, die jedoch alle olfaktorisch interessante Eigenschaften aufweisen.

Hydriert man in Gegenwart von Ruthenium beispielsweise bei einem Wasserstoffdruck von 50 bar und einer Temperatur von 120° C, so erhält man ein Isomerenverhältnis von A : B : C : D = 68 : 29 : 2 : 1 der im folgenden Schema dargestellten Isomeren. Hydriert man bei 50 bar Wasserstoffdruck und einer Temperatur von 200° C, so beträgt das Verhältnis A : B : C : D = 52 : 24 : 16 : 8. Die Isomerenverhältnisse wurden jeweils NMR-spektroskopisch bestimmt ($^{1}$H- und $^{13}$-C-NMR-Spektren).

Schema

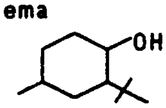

A

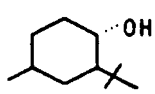

B

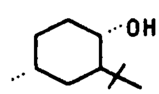

C

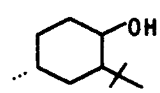

D

Das auf diese Weise erhaltene Diastereomerengemisch von I stellt eine farblose Flüssigkeit von erdig-holzigem, Vetiver-artigem Geruch dar.

Strukturell verwandte Verbindungen, die auf dem Riechstoff-Gebiet bereits Verwendung finden sind 1) d,l-Menthol und 2) 2-tert.-Butyl-cyclohexanol:

1) d,l-Menthol (2-Isopropyl-5-methyl-cyclohexanol), von dem vier unterschiedliche d,l-Paare unterschieden werden können, nämlich

- a) das d,l-Menthol, bei dem alle drei Substituenten in äquatorialer Lage sind,
- b) das d,l-Neomenthol, bei dem nur die Hydroxy-Gruppe in axialer Lage ist,
- c) das d,l-Isomenthol, bei dem nur die Methyl-Gruppe in axialer Lage ist und
- d) das d,l-Neoisomenthol, bei dem Hydroxy- und Methyl-Gruppe in axialer Lage sind.

Von allen vier Racematen besitzen d- und l-Menthol die bei weitem größte wirtschaftliche Bedeutung.

Es existieren extreme Unterschiede zwischen den olfaktorischen und organoleptischen Eigenschaften von d- und l-Menthol. Während das l-Isomere einen erfrischenden Geruch aufweist, der an Pfefferminze erinnert, weist d-Menthol eine holzige Kampfer-Note auf.

2) 2-tert.-Butyl-cyclohexanol weist eine starke, kampferähnliche minzige Note auf.

Es war daher überraschend, daß I eine völlig andere Geruchsnote aufweist.

Von besonderer Bedeutung ist, daß I aufgrund seines intensiven Geruches bereits bei sehr geringer Dosierung in Kompositionen mit holziger Note eine deutliche Akzentuierung sowie Ausdruck und Strahlkraft einbringt.

Erfindungsgemäß können einzelne Diastereomere und Diastereomerengemische eingesetzt werden, wie sie bei der beschriebenen Hydrierung erhalten werden.

Seine ausgezeichnete Stabilität im sauren, neutralen sowie im alkalischen Medium eröffnet ein breites Anwendungsfeld für den erfindungsgemäßen Riechstoff.

I läßt sich sehr gut mit den üblichen Parfüminhaltsstoffen und anderen Riechstoffen zu neuartigen Kompositionen kombinieren; der Anteil in den Riechstoffkompositionen beträgt im allgemeinen 0,1 - 50 Gewichtsprozent. Derartige Kompositionen können zur Parfümierung von kosmetischen Präparaten wie Cremes, Lotionen, Duftwässern, Aerosolen, Toilettenseifen, Mundpflegemitteln, für die Haarkosmetik sowie in der Extrait-Parfümerie verwendet werden. Weiterhin können Sie zur Geruchsverbesserung von technischen Produkten wie Wasch- und Reinigungsmitteln oder Weichspülern verwendet werden.

Der erfindungsgemäße Riechstoff kann einzeln und insbesondere im Gemisch mit anderen Riechstoffen eingesetzt werden.

Im folgenden wird die Erfindung durch Aufzeigen eines Herstellverfahrens, eines Anwendungsbeispiels sowie eines Stabilitätstests erläutert.

Beispiel 1
Herstellung von 2-tert.-Butyl-4-methyl-cyclohexanol

In einem Autoklaven wurde eine Mischung aus 400 g (2,44 Mol) 2-tert.-Butyl-4-methyl-phenol, 400 ml Dioxan sowie 1 g Rutheniumhydroxid vorgelegt und bei einer Temperatur von 120 °C und einem Wasserstoffdruck von 50 bar bis zur Druckkonstanz hydriert (Gesamthydrierzeit ca. 3 1/2 Stunden). Nach Abtrennung des Katalysators wurde das Dioxan abdestilliert und der Rückstand bei 0,01 mbar fraktioniert. Nach einer kleinen Vorlauf-Fraktion (15 g vom Kp. bis 60 °C/0,01 mbar) destillierten bei einer Temperatur von 80 bis 85 °C/0,01 mbar 401 g (2,36 Mol, entsprechend einer Ausbeute von 96 % der Theorie) 2-tert.-Butyl-4-methyl-cyclohexanol über, die nach dem Abkühlen zu einer halbfesten Masse erstarrten. Nach $^{13}$C-NMR-spektroskopischen Daten konnten sie als ein Diastereomerengemisch folgender Zusamensetzung identifiziert werden:

A : B : C • D = 68 : 29 : 2 : 1

Schema

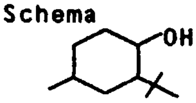
A

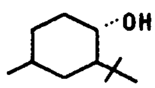
B

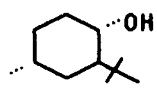
C

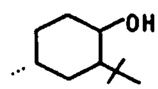
D

Das Diastereomerengemisch ($n_D^{25}$ 1.4679) wies eine hochinteressante intensive erdigholzige Vetiver-Note auf.

Je nach angewendeter Reaktionstemperatur und Wasserstoffdrücken gelangte man zu unterschiedlichen Diastereomerenverhältnissen an A, B, C, und D, wie in der folgenden Tabelle veranschaulicht ist.

Tabelle

| Beispiel | Temperatur [°C] | Wasserstoffdruck [bar] | Diastereomeren-Verhältnis | | | |
|---|---|---|---|---|---|---|
| | | | A | B | C | D |
| 1 a | 120 | 50 | 68 | 29 | 2 | 1 |
| 1 b | 160 | 50 | 61 | 28 | 9 | 2 |
| 1 c | 180 | 50 | 58 | 23 | 14 | 5 |
| 1 d | 200 | 50 | 53 | 24 | 16 | 7 |

Beispiel 2
Anwendung von I zur Abrundung einer Riechstoffkomposition

Parfümöl (Chypre-Typ):

| Bestandteile | Gehalt [Gew.-Teile] a | b |
|---|---|---|
| Citronellol | 40 | 40 |
| Dihydrorosenoxid | 10 | 10 |
| Phenylethylacetat | 20 | 20 |
| Isoeugenol | 5 | 5 |
| Coumarin | 15 | 15 |
| Bergamottöl | 80 | 80 |
| Fixolide® NP | 25 | 25 |
| Pentylcyclopentanon | 5 | 5 |
| Hedione® | 20 | 20 |
| Benzylacetat | 40 | 40 |
| Aldehyd C 10 10 % | 10 | 10 |
| Aldehyd C 11 (Undecylen) 10 % | 20 | 20 |
| Methylionon gamma | 80 | 80 |
| Vetiverylacetat | 50 | 50 |
| Ambrox® 10 % in Diethylphthalat | 5 | 5 |
| Jasmorange*/Anthranilate | 10 | 10 |

| Bestandteile | Gehalt [Gew.-Teile] a | b |
|---|---|---|
| Styrallylacetat | 10 | 10 |
| Phenylacetaldehyddimethylacetal | 10 | 10 |
| Tetrahydrolinalool | 40 | 40 |
| Hydroxycitronellal | 40 | 40 |
| Ylang Ylang Öl | 5 | 5 |
| Petitgrain Öl | 30 | 30 |
| Labdanym res. | 10 | 10 |
| Mousse de Chene abs. | 25 | 25 |
| Patchouli Öl | 30 | 30 |
| Linalylacetat | 50 | 50 |
| Aldehyd C 14 sog. 10 % | 30 | 30 |
| Dimethylbenzylcarbinylacetat | 10 | 10 |
| Phenylethylalkohol extra | 100 | 100 |
| Sandelether* | 40 | 40 |
| Dipropylenglykol (DPG) | 135 | 95 |
| 2-tert.-Butyl-4-methyl-cyclohexanol | - | 40 |
| | 1000 | 1000 |

* = angemeldete Warenzeichen

Das Parfümöl der Zusammensetzung a ist ein Chypre-Komplex mit einer deutlichen Note von Vetiver. Setzt man dem Komplex a 40 Teile (4 Gew.-%) 2-tert.-Butyl-4-methyl-cyclohexanol anstelle von 40 Teilen

DPG zu, so wirkt die ganze Komposition intensiver; der holzige Komplex wird deutlicher akzentuiert, die Vetiver-Note wird deutlich unterstrichen und die Komposition wirkt angenehm abgerundet, zeichnet sich durch mehr Charakter und Fülle aus.

Beispiel 3
Stabilitätstests

2-tert.-Butyl-4-methyl-cyclohexanol wurde 30 Tage in saurem, neutralen und basischem Medium bei 40 °C gelagert. Dazu wurden jeweils 0.5 Gew.-%ige Lösungen von 2-tert.-Butyl-4-methyl-cyclohexanol in 60 Gew.-%igem, wäßrigem Ethanol bereitet und diese teilweise mit 1 n HCl oder 1 n NaOH auf einen pH-Wert von 1,7 bzw. 14 gebracht. Unter den oben genannten Bedingungen war I nach dünnschichtchromatographischer Untersuchung in allen Testlösungen stabil. Es konnten auch keine Farb- oder Geruchsveränderungen festgestellt werden.

**Patentansprüche**

1. Verwendung von 2-tert.-Butyl-4-methyl-cyclohexanol als Riechstoff.

2. Riechstoffkompositionen, gekennzeichnet durch einen Gehalt an 2-tert.-Butyl-4-methyl-cyclohexanol.

3. Riechstoffkompositionen nach Anspruch 2, dadurch gekennzeichnet, daß sie 2-tert.-Butyl-4-methylcyclohexanol in einer Menge von 0,1 bis 50 Gewichtsprozent bezogen auf die gesamte Komposition enthalten.

**Claims**

1. Use of 2-tert-butyl-4-methylcyclohexanol as a scent.

2. A scent composition which contains 2-tert-butyl-4-methylcyclohexanol.

3. A scent composition as claimed in claim 2, which contains 2-tert-butyl-4-methylcyclohexanol in an amount of from 0.1 to 50% by weight, based on the total composition.

**Revendications**

1. Utilisation du 2-tert.-butyl-4-méthyl-cyclohexanol comme parfum.

2. Compositions parfumées, caractérisées en ce qu'elles contiennent du 2-tert.-butyl-4-méthyl-cyclohexanol.

3. Compositions parfumées selon la revendication 2, caractérisées en ce qu'elles contiennent du 2-tert.-butyl-4-méthyl-cyclohexanol en quantités comprises entre 0,1 et 50% en poids par rapport à la composition totale.